# EUROPEAN PATENT APPLICATION

(11) **EP 4 678 631 A1**
(43) Date of publication of application: **14.01.2026**
(21) Application number: 24766906.2
(22) Date of filing: 22.02.2024
(51) Int. Cl.: C07C 319/02, C07B 61/00, C07C 321/04, C07C 321/06, C07C 321/10, C07C 321/22

(54) **THIOL PRODUCTION METHOD**

(30) Priority: 07.03.2023 JP 2023034877
(71) Applicant: DIC Corporation, Tokyo 174-8520 (JP); Kyushu University, National University Corporation, Nishi-ku Fukuoka-shi Fukuoka 819-0395 (JP)
(72) Inventor: MATSUEDA, Hironobu, Kamisu-shi, Ibaraki 314-0193 (JP); XU, Ke, Fukuoka-shi, Fukuoka 819-0395 (JP); YAMAMOTO, Eiji, Fukuoka-shi, Fukuoka 819-0395 (JP); TOKUNAGA, Makoto, Fukuoka-shi, Fukuoka 819-0395 (JP); MURAYAMA, Haruno, Fukuoka-shi, Fukuoka 819-0395 (JP)
(74) Representative: Kraus & Lederer PartGmbB
(86) International application number: PCT/JP2024/006567
(87) International publication number: WO 2024/185535

(57) **Abstract**

In a thiol production method according to an embodiment, an alkene (1) or a derivative thereof is reacted with sulfur in the presence of hydrogen and a metallic element to produce thiol (2).

## Description

### Technical Field

The present invention relates to a thiol production method.

The present application claims priority based on Patent Application No. 2023-034877 filed in Japan on March 7, 2023, the contents of which are hereby incorporated herein by reference.

### Background Art

Thiols are used in various applications including gas odorants, fragrances, and ligands. Examples of the gas odorants, fragrances, and ligands are thiophenol, 3-mercaptohexanol, and tert-butylthiol, respectively.

Examples of typical methods for producing thiol include: (1) a synthesis method employing substitution reaction (SN₂ reaction) for interacting reactants in a single step (NPL 1); (2) a synthesis method of thiols from alcohol material using hydrogen sulfide (NPL 2); (3) a synthesis method of thiols from alkene using hydrogen sulfide (NPL 3); (4) a synthesis method of thiols from alkene using ultraviolet or a platinum electrode (NPL 4); and (5) a synthesis method of thiols using sulfur and a carbonyl compound (NPL 5).

### Citation List

### Non Patent Literature

NPL 1: J. Org. Chem. 1968, 33, pp. 1275-1276
NPL 2: Catalysis Lett. 2000, 64, 197-200
NPL 3: Ind. Eng. Chem. 1948, 40, No. 12, pp. 2308-2313.
NPL 4: J. Org. Chem. 1942, 07, 6, pp. 472-476
NPL 5: Nippon kagaku kaishi, 1987, 7, pp. 1502-1504

### Summary of Invention

### Technical Problem

However, the synthesis method disclosed in the above (1) is low in atom efficiency and produces a large amount of wastes as by-products. The synthesis method disclosed in the above (2), which requires stringent conditions (e.g., high reaction temperature), is difficult for use in actual production. The synthesis method disclosed in the above (3), which entails the use of highly toxic hydrogen sulfide, requires complicated handling in terms of equipment management and the like. Further, the synthesis method disclosed in (4) requires an ultraviolet radiator or expensive platinum electrode, resulting in increase in production cost. The synthesis method disclosed in the above (5), which requires the use of CO gas and expensive organic base, results in complicated material handling and increase in production cost, as in the above (3) and (4).

An object of the present invention is to provide a thiol production method capable of easily manufacturing thiol with high atom efficiency, easily handling the material, and achieving reduction in production cost. Solution to Problem

In order to achieve the above object, the present inventors have found, after vigorous studies, that various thiols can be easily produced by a reaction of sulfur and hydrogen to a carbon atom in the second position of an alkene, which has a double bond at a terminal end or a part thereof, in the presence of a catalyst including a metallic element. In particular, it has been found that, with the use of (gas) hydrogen that is increasingly easily available as a next-generation fuel, various thiols can be produced without the use of difficult-to-handle hydrogen sulfide gas or expensive organic base and without the use of ultraviolet radiator, platinum electrode, and the like.

Specifically, the present invention provides the following solutions.
[1] A thiol production method including reacting an alkene (1) or a derivative thereof with sulfur in the presence of hydrogen and a metallic element to obtain a thiol (2).
[2] The thiol production method according to the above [1], in which a pressure of supplied hydrogen is in a range from 0.1 MPa to 10 MPa inclusive when the alkene (1) or the derivative thereof is reacted with sulfur.
[3] The thiol production method according to the above [1] or [2], in which a heating temperature is in a range from 100 °C to 200 °C inclusive when the alkene (1) or the derivative thereof is reacted with sulfur in the presence of hydrogen.
[4] The thiol production method according to any one of the above [1] to [3], in which the metallic element is one or more metallic elements selected from Group 6 to Group 11.
[5] The thiol production method according to the above [4], in which the metallic element is a metallic element constituting a metal oxide or a metal sulfide.
[6] The thiol production method according to any one of the above [1] to [5], in which an added amount of the metallic element with respect to the alkene (1) or the derivative thereof is in a range from 0.1 mol% to 10 mol% inclusive.
[7] The thiol production method according to any one of the above [1] to [6], in which the alkene (1) or the derivative thereof is reacted with sulfur in the presence of hydrogen, the metallic element, and zeolite.
[8] The thiol production method according to the above [7], in which the zeolite is basic.
[9] The thiol production method according to the above [7] or [8], in which the zeolite has a type X or type A pore structure.
[10] The thiol production method according to any one of the above [7] to [9], in which an added amount of the zeolite is in a range from 2.0 parts by mass to 25 parts by mass inclusive with respect to 100 parts by mass of the alkene (1).
[11] The thiol production method according to any one of the above [1] to [10], in which the alkene (1) is represented by R¹R²C=CH₂ ... (A), where R¹ is an alkyl group, R² is a hydrogen atom or an alkyl group, and a total number of carbon atoms of R¹ and R² is 2 to 20.
[12] The thiol production method according to the above [11], in which the total number of the carbon atoms of R¹ and R² in the above general formula (A) is 2 to 16. Advantageous Effects of Invention

According to the present invention, a thiol production method capable of easily producing thiol with high atom efficiency, easily handling the material, and reducing production cost can be provided.

### Brief Description of Drawings

FIG. 1 is a schematic diagram illustrating a reaction in a thiol production method according to an embodiment of the present invention.
FIG. 2 is a schematic diagram illustrating a reaction in a method for producing a thiol (2a) in Example 1.
FIG. 3 is a graph illustrating a temporal change in a yield of the thiol (2a) and a total yield of dialkyl monosulfides (3a) to (5a) in Example 16.
FIG. 4 is a graph illustrating spectra obtained by XRD measurements on CoS, CoS₂ before being used in Examples 2 and 3, cobalt-silicon composite oxides (CoOₓ-SiO_{y}) before being used in Example 6, CoOₓ-SiO_{y} after being used in Example 6, and α-Co₂SiO₄ before being used in Example 9.
FIG. 5 is a diagram illustrating elemental mapping images obtained by EDS measurements on CoOₓ-SiO_{y} before being used in Example 6.
FIG. 6 is a diagram illustrating elemental mapping images obtained by EDS measurements on CoOₓ-SiO_{y} after being used in Example 6.
FIG. 7 is a diagram illustrating structural formulae of compounds (1b) to (1s) used in Examples 18 to 35.
FIG. 8 is a diagram illustrating structural formulae of thiols (2b) to (2s) synthesized in Examples 18 to 35. Description of Embodiments

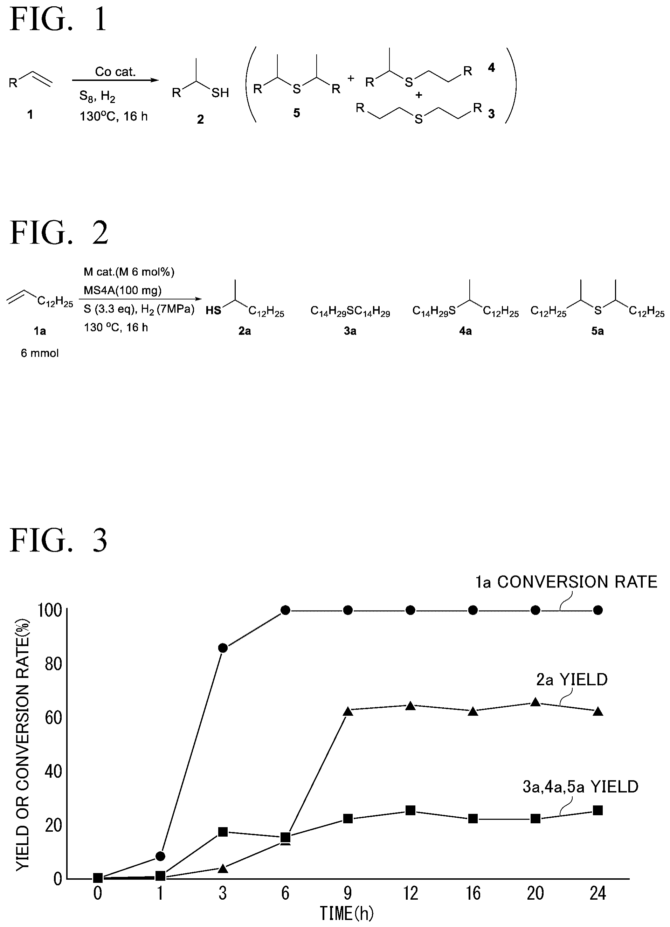

According to a thiol production method according to an embodiment of the present invention, an alkene (1) or a derivative thereof is reacted with sulfur in the presence of hydrogen and a metallic element to obtain a thiol (2). FIG. 1 illustrates the reaction in the thiol production method according to the present embodiment. The reaction conditions illustrated in FIG. 1 such as catalyst type, reaction temperature, and heating time, are merely exemplary and can be changed depending on the type of the material and the like.

### (Alkene (1))

Examples of the alkene (1) used in the above process, which are not specifically limited as long as a double bond is present at a terminal end or a part of the alkene (1), include an acyclic olefin (1A), a cyclic olefin (1B) or a derivative thereof.

The acyclic olefin (1A) used in the present embodiment is represented by, for example, R¹R²C=CH₂ ... (A), where R¹ is an alkyl group, R² is a hydrogen atom or an alkyl group, and a total number of carbon atoms of R¹ and R² is 2 to 20. In order to provide an industrial material suited to properties of sulfur, among the examples of the alkene (1), an alkene whose total number of carbon atoms of R¹ and R² is 2 to 18 is preferable, and an alkene whose total number of carbon atoms of R¹ and R² is 2 to 16 is more preferable. Examples of the acyclic olefin (1A) to be used in the present embodiment include compounds (1a), (1b), and (1h) described below (see FIG. 9).

The above acyclic olefin (1A) optionally has other functional group(s) such as an aryl group, an alkoxy group, a hydroxy group, a cycloalkyl group, and a silylalkyl group. When the above acyclic olefin (1A) further has an aryl group, examples of the acyclic olefin (1A) include later-described compounds (1f), (1i), and (1l) to (1r) (see FIG. 9). When the above acyclic olefin (1A) further has an alkoxy group, examples of the acyclic olefin (1A) include a later-described compound (1s). When the above acyclic olefin (1A) further has a hydroxy group, examples of the acyclic olefin (1A) include later-described compounds (1j) and (1k). When the above acyclic olefin (1A) further has a cycloalkyl group, examples of the acyclic olefin (1A) include later-described compounds (1d) and (1e). When the above acyclic olefin (1A) further has a silylalkyl group, examples of the acyclic olefin (1A) include later-described compound (1c).

The cyclic olefin (1B) used in the present embodiment is, for example, represented by CₙH₂ₙ₋₂ ... (B), where n≥3. Examples of the cyclic olefin (1B) include later-described compound (1g).

In the manufacturing method of the present embodiment, an alkene other than the alkene (1) is optionally used in combination with the alkene (1) as long as an effect of the invention is not impaired. In other words, the material used in the manufacturing method of the present embodiment optionally includes the alkene (1) and the alkene other than the alkene (1).

### (Sulfur)

Sulfur to be used in the present embodiment, whose nature is not specifically limited, is optionally in a form of a solid (e.g., small masses, flakes, and powder) or in form of a melt (liquid). Among the above, for the convenience of a charging process in a large scale production, melt sulfur is preferable.

In the above process, in view of solubility, the ratio of used alkene (1) to sulfur is preferably 1.0 to 5.0 mol in terms of S element (1.0 to 5.0 molar equivalents in terms of S equivalent: 0.125 to 0.625 molar equivalents in terms of S₈) per 1 mol olefin (a), more preferably 1.0 to 4.0 mol (1.0 to 4.0 molar equivalents in terms of S equivalent; 0.125 to 0.5 molar equivalents in terms of S₈), and 1.0 to 3.5 mol (1.0 to 3.5 molar equivalents in terms of S equivalent; 0.125 to 0.438 molar equivalents in terms of S₈).

### (Hydrogen)

Typical example of hydrogen used in the present embodiment is hydrogen gas. Hydrogen gas is widely distributed and thus is more easily available than hydrogen sulfide. In addition, hydrogen gas, which is, although flammable, non-toxic and odorless, is more easily handled as compared with hydrogen sulfide. Hydrogen gas is not limited but can be, for example, 99.99% or more, 99.999% or more, or 99.9999% or more.

Hydrogen gas, which can be produced through non-limited process, may be commercially available hydrogen gas or may be produced by a reforming method, in which hydrogen is produced by reacting a hydrocarbon (e.g., methane) with water vapor, or an electrolysis method, in which water is electrolyzed to produce hydrogen.

When the alkene (1) is reacted with sulfur in the presence of hydrogen, the pressure of supplied hydrogen is preferably in a range from 0.1 MPa to 10.0 MPa inclusive, more preferably in a range from 1.0 MPa to 9.0 MPa inclusive, and even more preferably in a range from 2.0 MPa to 8.0 MPa inclusive. The yield of the resulting thiol (2) can be increased by setting the pressure of the supplied hydrogen in the range from 0.1 MPa to 10.0 MPa inclusive.

The heating temperature for the reaction of the alkene (1) with sulfur in the presence of hydrogen is preferably in a range from 100 °C to 200 °C inclusive, more preferably in a range from 100 °C to 160 °C inclusive, even more preferably in a range from 100 °C to 140 °C inclusive, and especially preferably in a range from 120 °C to 140 °C inclusive. By setting the heating temperature during the reaction in the range from 100 °C to 200 °C inclusive, the yield of the resulting thiol (2) can be increased while reducing the reaction time to improve productivity.

The thiol (2), which is produced by reacting the alkene (1) with sulfur in the presence of hydrogen in the above process, is optionally produced by reacting the alkene (1) with sulfur in the presence of hydrogen and a metallic element. The yield of the resulting thiol (2) can be increased by a contact reaction of the alkene (1) with sulfur by using the metallic element as a catalyst in the presence of hydrogen.

### (Metallic Element)

The metallic element used in the present embodiment is preferably one or more metallic elements selected from Group 6 to Group 11. Examples of such metallic elements include the following.
Group 6: Chromium (Cr), Molybdenum (Mo), Tungsten (W)
Group 7: Manganese (Mn), Technetium (Tc), Rhenium (Re)
Group 8: Iron (Fe), Ruthenium (Ru), Osmium (Os)
Group 9: Cobalt (Co), Rhodium (Rh), Iridium (Ir)
Group 10: Nickel (Ni), Palladium (Pd), Platinum (Pt)
Group 11: Copper (Cu), Silver (Ag), Gold (Au)

The above metallic elements are preferably metallic elements that constitute, for example, metal oxides, metal sulfides, or metal carbonates. When the above metallic element is, for example, a metallic element constituting a metal oxide, the alkene (1) and sulfur are reacted in the presence of hydrogen and the above metal oxide. When the above metallic element is a metallic element constituting a metal sulfide, the alkene (1) and sulfur are reacted in the presence of hydrogen and the above metal sulfide. The valence of the metallic element constituting the metal oxide or metal sulfide is not particularly limited but can take various values such as +1 and +2.

When the above metallic element is a metallic element constituting a metal oxide, examples of the metal oxide include, but are not limited to, the following.
Group 6 metal oxides: CrO₃, MoO₃, WO₃
Group 7 metal oxides: Mn₂O₃, Tc₂O₇, ReO₃
Group 8 metal oxides: Fe₂O₃, RuO₂, OsO₄
Group 9 metal oxides: CoO, Rh₂O₃, IrO₂
Group 10 metal oxides: NiO, PdO, PtO₂
Group 11 metal oxides: CuO, Ag₂O, Au₂O₃

When the above metallic element is a metallic element constituting a metal sulfide, examples of the metal sulfide include, but are not limited to, the following.
Group 6 metal sulfides: Cr₂S₃, MoS₂, WS₂
Group 7 metal sulfides: MnS, ReS₂
Group 8 metal sulfides: FeS, RuS₂, OsS₂
Group 9 metal sulfides: CoS, Rh₂S₃, IrS₂
Group 10 metal sulfides: Ni₃S₂, PdS, PtS
Group 11 metal sulfides: CuS, Ag₂S, Au₂S

When the above metallic element is, for example, cobalt (Co), cobalt oxide and cobalt sulfide can be used as the metal oxide and the metal sulfide, respectively. When cobalt oxide is used as the metal oxide, the alkene (1) is reacted with sulfur in the presence of hydrogen and cobalt oxide. Examples of the usable cobalt oxide include, but not limited to, CoO, Co₂O₃, and Co₃O₄. When cobalt sulfide is used as the metal sulfide, the alkene (1) is reacted with sulfur in the presence of hydrogen and cobalt sulfide. Examples of the usable cobalt sulfide include, but not limited to, CoS, Co₉S₈, CoS₂, and Co₃S₄. Among the above, in order to increase the yield of dialkyl polysulfide (A), cobalt oxide, especially Co₃O₄ is preferable.

When the above metallic element is a metallic element constituting a metal oxide, the metal oxide optionally includes a plurality of metallic elements selected from Group 6 to Group 11. In this case, examples of the usable metal oxide include NiCo₂O₄. When the above metallic element is a metallic element constituting a metal oxide, the metal oxide optionally includes a metallic element selected from Group 6 to Group 11 and an element other than the metallic element. Examples of the element other than the metallic element include silicon (Si). In this case, examples of the usable metal oxide include CoOₓ-SiO_{y} (e.g., Co₂SiO₄ and α-Co₂SiO₄) and NiOₓ-SiO_{y} (e.g., Ni₂SiO₄) .

When the above metallic element is a metallic element constituting a metallic carbonate, examples of the usable metallic carbonate include CoCO₃.

The added amount of the metallic element with respect to the alkene (1) is preferably in a range from 0.1 mol% to 10 mol% inclusive, more preferably in a range from 1.0 mol% to 10 mol% inclusive, and even more preferably in a range from 2.0 mol% to 7.0 mol% inclusive. When the added amount of the metallic element with respect to the alkene (1) is in the range from 0.1 mol% to 10 mol% inclusive, the reaction of the alkene (1) and sulfur in the presence of hydrogen is promoted to increase the yield of the resulting thiol (2).

### (Zeolite)

The thiol (2), which is obtained by reacting the alkene (1) with sulfur in the presence of hydrogen and the metallic element in the manufacturing method of the present embodiment, is optionally produced by reacting the alkene (1) with sulfur in the presence of hydrogen, the metallic element, and zeolite. The yield of the resulting thiol (2) can be further increased by a contact reaction of the alkene (1) with sulfur in the presence of hydrogen using the metallic element and zeolite as catalysts.

Zeolite is a crystalline aluminosilicate, which has a skeletal structure of regularly linked silica and alumina, contains cations as ion exchange sites in the pore structure. The pore structure defined by the skeletal structure of zeolite includes, but not limited to, LTA (type A), FER (ferrierite), MWW (MCM-22), MFI (ZSM-5), MOR (mordenite), LTL (type L), FAU (type Y, type X), and BEA (type beta). Among the above, it is preferable that zeolite has a type X or type A pore structure in terms of availability and economic efficiency.

The above zeolite is preferably basic. Examples of the basic zeolite include zeolites containing alkali metal or alkaline earth metal cations. Examples of the alkali metal include sodium (Na) and potassium (K). Examples of the alkaline earth metal include magnesium (Mg) and calcium (Ca).

In order to increase the yield of the thiol (2), the above zeolite is preferably of a type Na-X, which has a skeletal structure of the type X and contains sodium ions, or of a type Na-A, which has a skeletal structure of the type A and contains sodium ions. Among the above, the type Na-A is more preferable.

The added amount of the above zeolite is preferably in a range from 2.0 parts by mass to 25 parts by mass inclusive with respect to 100 parts by mass of the alkene (1), more preferably in a range from 2.0 parts by mass to 15 parts by mass inclusive, further preferably in a range from 3.0 parts by mass to 10 parts by mass inclusive, and especially preferably in a range from 5.0 parts by mass to 9.0 parts by mass inclusive. When the added amount of the above zeolite is in the range from 2.0 parts by mass to 25 parts by mass inclusive with respect to 100 parts by mass of the alkene (1), the yield at the time of first recycling can be maintained at a high value.

The thiol (2) is obtained by the manufacturing method of the present embodiment. In the present manufacturing method, one or more other compounds other than thiol (2) is optionally obtained in addition to the thiol (2). Examples of other compounds include dialkyl monosulfides. The content (yield) of each of the thiol (2) and dialkyl monosulfides whose numbers of carbon atoms are different can be determined by a peak area of a chart obtained by gas chromatography (hereinafter also referred to as "GC") measurement.

The thiol (2) can be efficiently produced by the manufacturing method of the present embodiment. The resulting thiol (2) is suitably usable, for example, as gas odorants, fragrances, or ligands.

### [Examples]

Examples of the invention will be described below. The scope of the present invention is not limited to the examples described below.

### (Example 1)

After charging, in an autoclave, a stirrer, 6 mmol of the compound (1a) (H₂C=C-C₁₂H₂₅), 0.4125 molar equivalents of sulfur (S₈) (3.3 molar equivalents as S element), 6 mol% of Co₃O₄ (manufactured by Sigma-Aldrich Co. LLC, nanopowder: 50 nm or less (TEM)), and 100 mg zeolite (manufactured by Sigma-Aldrich Co. LLC, Molecular Sieve 4A, type Na-A), hydrogen was pressure-charged at a pressure up to 7.0 MPa. While stirring at 800 rpm with a magnetic stirrer, the autoclave was heated to 130 °C, at which the reaction was carried out for 16 hours. Then, after cooling to room temperature and opening a pressure valve, 20 mg tridecane (1.7 parts by mass with respect to 100 parts by mass of the compound (1a)) was added to the reaction solution as an internal standard. Then, air was blown in to remove any residual hydrogen sulfide. After removing unreacted sulfur and catalyst by a centrifugal separator, the thiol (2a) was obtained. The reaction in the method for producing the thiol (2a) in Example 1 is illustrated in FIG. 2.

### (Example 2)

The thiol (2) was obtained in the same manner as in Example 1 except that the type of the metal catalyst was changed from Co₃O₄ to CoS (manufactured by Strem Chemicals, Inc.).

### (Example 3)

The thiol (2a) was obtained in the same manner as in Example 1 except that the type of the metal catalyst was changed from Co₃O₄ to CoS₂ (manufactured by Alfa Aesar).

### (Example 4)

The thiol (2a) was obtained in the same manner as in Example 1 except that the type of the metal catalyst was changed from Co₃O₄ to nickel-cobalt composite oxide (NiOₓ-CoO_{y}). The nickel-cobalt composite oxide was prepared as follows.

Nickel acetate tetrahydrate (manufactured by KISHIDA CHEMICAL CO., LTD., 2.48 g, 10 mmol) and cobalt nitrate nonahydrate (manufactured by FUJIFILM Wako Pure Chemical Corporation, 5.82 g, 20 mmol) were dissolved in 150 mL distilled water, into which a solution of ammonium carbonate (manufactured by FUJIFILM Wako Pure Chemical Corporation, 2.88 g, 30 mmol) dissolved in 50 mL distilled water was added dropwise over 5 minutes while stirring, and was left still at room temperature for 3 hours. After the reaction, the resulting precipitate was filtered off, washed twice with 50 mL distilled water, and dried in air at 70 °C for 15 hours to obtain a black solid. The solid was then ground in a mortar and sieved to collect black powder of a fineness of 125 µm or less. The powder was then transferred to a calcination dish for calcination. The calcination was conducted under temperature conditions of raising the temperature to 300 °C in an hour (temperature rise program: 5 °C/min) and maintaining the temperature at 300 °C for 6 hours. After calcination, the resultant substance was cooled to room temperature to collect black powder (2.16 g). The contents of cobalt and nickel, which were evaluated using a microwave plasma atomic emission spectrometer (MP-AES), were 38 mass% and 11 mass%, respectively.

### (Example 5)

The thiol (2a) was obtained in the same manner as in Example 1 except that the type of the metal catalyst was changed from Co₃O₄ to NiO (manufactured by Sigma-Aldrich Co. LLC).

### (Example 6)

The thiol (2a) was obtained in the same manner as in Example 1 except that the type of the metal catalyst was changed from Co₃O₄ to cobalt-silicon composite oxide (CoOₓ-SiO_{y}). The cobalt-silicon composite oxide was prepared as follows.

Cobalt nitrate hexahydrate (FUJIFILM Wako Pure Chemical Corporation, 2.91 g, 10 mmol) was dissolved in 90 mL distilled water, into which a solution of sodium silicate (manufactured by Sigma-Aldrich Co. LLC, 1.22 g, 10 mmol) dissolved in 10 mL distilled water was added dropwise over 3 minutes while stirring, and was left still at room temperature for 3 hours. After the reaction, the resulting precipitate was filtered off, washed twice with 50 mL of distilled water, and dried in air at 70 °C for 14 hours to obtain a purple solid. The solid was then ground in a mortar and sieved to collect pink powder (1.6 g) of a fineness of 125 µm or less. The cobalt content, which was evaluated by MP-AES, was 30 mass%.

### (Example 7)

The thiol (2a) was obtained in the same manner as in Example 1 except that the type of the metal catalyst was changed from Co₃O₄ to CoCO₃ (manufactured by FUJIFILM Wako Pure Chemical Corporation).

### (Example 8)

The thiol (2a) was obtained in the same manner as in Example 1 except that the type of the metal catalyst was changed from Co₃O₄ to nickel-silicon composite oxide (NiOₓ-SiO_{y}). NiOₓ-SiO_{y} was prepared as follows.

Nickel acetate tetrahydrate (manufactured by KISHIDA CHEMICAL CO., LTD., 4.98 g, 20 mmol) was dissolved in 90 mL distilled water, into which a solution of sodium silicate (manufactured by Sigma-Aldrich Co. LLC, 2.44 g, 20 mmol) dissolved in 10 mL distilled water was added dropwise over 3 minutes while stirring, and was left still at room temperature for 3 hours. After the reaction, the resulting precipitate was filtered off, washed twice with 50 mL distilled water, and dried in air at 70 °C for 14 hours to obtain a purple solid. The solid was then ground in a mortar and sieved to collect green powder (3.97 g) of a fineness of 125 µm or less. Nickel content, which was evaluated by MP-AES, was 29 mass%.

### (Example 9)

The thiol (2a) was obtained in the same manner as in Example 1 except that the type of the metal catalyst was changed from Co₃O₄ to α-Co₂SiO₄. The α-Co₂SiO₄ was prepared as follows. After being transferred to a quartz or alumina board, CoOₓ-SiO_{y} (1.1 g) was charged into a vacuum calcination furnace with a hydrogen generator, where hydrogen displacement was performed three times. Then, the temperature was raised to 1000 °C in 3 hours and 18 minutes (temperature rise program: 5 °C/min) in a hydrogen atmosphere. Then, the temperature was maintained in a hydrogen atmosphere for 3 hours. After calcination, the temperature was cooled to room temperature and purple powder (1 g) was collected. The cobalt content, which was evaluated by MP-AES, was 25 mass%.

The thiol (2) obtained in Examples 1 to 9 was measured and evaluated as follows.

### [Determination of Thiol Yield]

The yields of the thiol (2a) and dialkyl monosulfides (3a) to (5a) were calculated based on respective peak areas in a chart of the thiol (2) and dialkyl monosulfides (3a) to (5a) obtained using a gas chromatograph (manufactured by Agilent Technologies, 6850 Series II) and a column (Agilent manufactured by Technologies, HP-1, 30 m, 0.32 mm diameter, 0.25 µm film thickness) under the following measurement conditions. The conversion rate was calculated based on the yield of the compound (1a). The measurements and evaluation results of Examples 1 to 9 are listed in Table 1.

### [GC Measurement Conditions]

### (Temperature Rise Program)

1. 40 °C, held for 5 min
2. Temperature raised to 240 °C at 10 °C/min
3. Held for 15 minutes after reaching 240 °C
   Various Settings of GC
   - Used gases: Nitrogen (carrier gas), hydrogen (for detector), air (for detector)
   - Inlet settings: Heater 200 °C, pressure 93 kPa, total flow (N₂) 24.5 mL/min, split ratio 5.3:1
   - Column settings: Pressure 93 kPa, flow rate (N₂) 3.6 mL/min, average linear velocity 52 cm/sec
   - Detector settings: Heater 250 °C, hydrogen flow rate 30.0 mL/min, air flow rate 250.0 mL/min, make-up flow rate (N₂) 10.0 mL/min
      (GC Retention Time of Standard Used for Identification of Each Compound)

   - Internal standard (tridecane): 13.5 min
   - Tetradecene: 14.9 min
   - Thiol: 18.8 min
   - Dialkylmonosulfanes (3a: 38.7 min, 4a: 36.0 min, 5a: 34.8 min)

**[Table 1]**

| Ex. | Catalyst | Catalyst amount (mol%) | Zeolite (mg) | Hydrogen pressure (MPa) | s equivalent | Temperature (°C) | Time (h) | Conversion rate (%) | 2a Yield (%) | 3a Yield (%) | 4 a Yield (%) | 5a Yield (%) |
|---|---|---|---|---|---|---|---|---|---|---|---|---|
| 1 | Co₃O₄ | 6 | 100 | 7 | 3.3 | 130 | 16 | 99 | 12 | 2 | 6 | 7 |
| 2 | CoS | 6 | 100 | 7 | 3.3 | 130 | 16 | 95 | 5 | 2 | 4 | 5 |
| 3 | COS₂ | 6 | 100 | 7 | 3.3 | 130 | 16 | 99 | 15 | 2 | 7 | 8 |
| 4 | NiOₓ-CoO_{y} | 6 | 100 | 7 | 3.3 | 130 | 16 | 99 | 21 | 3 | 3 | 2 |
| 5 | NiO | 6 | 100 | 7 | 3.3 | 130 | 16 | 98 | 57 | 2 | 7 | 7 |
| 6 | CoOₓ-SiO_{y} | 6 | 100 | 7 | 3.3 | 130 | 16 | 99 | 63 | 2 | 5 | 5 |
| 7 | CoCO₃ | 6 | 100 | 7 | 3.3 | 130 | 16 | 99 | 62 | 2 | 5 | 4 |
| 8 | NiOₓ-SiO_{y} | 6 | 100 | 7 | 3.3 | 130 | 16 | 98 | 6 | 6 | 5 | 2 |
| 9 | α-Co₂SiO₄ | 6 | 100 | 7 | 3.3 | 130 | 16 | 96 | 3 | 2 | 4 | 5 |

The results in Table 1 indicate that, in any of Examples 1 to 9, the thiol (2a) can be produced at 3 to 63% yield by reacting the compound (1a) (alkene (1)) and powdered sulfur in the presence of hydrogen, one of Co₃O₄, CoS, CoS₂, NiCo₂O₄, NiO, Co₂SiO₄, CoCO₃, Ni₂SiO₄ and Co₂SiO₄ and zeolite. It was found, in particular, in Examples 5 to 7 that the reaction of the compound (1a) with sulfur in the presence of hydrogen, one of NiO, Co₂SiO₄, and CoCO₃, and zeolite resulted in a total yield of the thiol (2a) exceeding 50%. The ratio of the yield of the thiol (2a) to the total yield of the thiol (2a) and dialkyl monosulfides (3a) to (5a) was greater than 20% in all of Examples 1 to 9.

### (Example 10)

The thiol (2a) was obtained in the same manner as in Example 6 except that the amount of powdered sulfur (S₈) was changed from 3.3 molar equivalents to 1.7 molar equivalents in terms of S element.

### (Example 11)

The thiol (2a) was obtained in the same manner as in Example 6 except that zeolite was not used.

### (Example 12)

The thiol (2a) was obtained in the same manner as in Example 6 except that zeolite was not used and the hydrogen pressure at the time of preparation was changed from 7.0 MPa to 5.0 MPa.

### (Example 13)

The thiol (2a) was obtained in the same manner as in Example 6 except that zeolite was not used and the hydrogen pressure at the time of preparation was changed from 7.0 MPa to 3.0 MPa.

### (Example 14)

The thiol (2a) was obtained in the same manner as in Example 6 except that zeolite was not used and the heating temperature of the autoclave (i.e., the reaction temperature) was changed from 130 °C to 120 °C.

### (Example 15)

The thiol (2a) was obtained in the same manner as in Example 6 except that zeolite was not used and the heating temperature of the autoclave (i.e., the reaction temperature) was changed from 130 °C to 140 °C.

### (Comparative Example 1)

The thiol (2a) was obtained in the same manner as in Example 6 except that no catalyst was used. The measurements and evaluation results of Examples 10 to 15 and Comparative Example 1 are listed in Table 2.

**[Table 2]**

| Ex. | Catalyst | Catalyst amount (mol%) | Zeolite (mg) | Hydrogen pressure (MPa) | S equivalent | Temperature (°C) | Time (h) | Conversion rate (%) | 2a Yield (%) | 3a Yield (%) | 4a Yield (%) | 5a Yield (%) |
|---|---|---|---|---|---|---|---|---|---|---|---|---|
| 6 | CoOₓ-SiO_{y} | 6 | 100 | 7 | 3.3 | 130 | 16 | 99 | 63 | 2 | 5 | 5 |
| 10 | CoOₓ-SiO_{y} | 6 | 100 | 7 | 1.7 | 130 | 16 | 84 | 27 | 3 | 7 | 8 |
| 11 | CoOₓ-SiO_{y} | 6 | - | 7 | 3.3 | 130 | 16 | 99 | 64 | 2 | 5 | 5 |
| 12 | CoOₓ-SiO_{y} | 6 | - | 5 | 3.3 | 130 | 16 | 99 | 54 | 3 | 4 | 4 |
| 13 | CoOₓ-SiO_{y} | 6 | - | 3 | 3.3 | 130 | 16 | 99 | 18 | 1 | 3 | 3 |
| 14 | CoOₓ-SiO_{y} | 6 | - | 7 | 3.3 | 120 | 16 | 99 | 55 | 2 | 5 | 5 |
| 15 | CoOₓ-SiO_{y} | 6 | - | 7 | 3.3 | 140 | 16 | 99 | 62 | 1 | 4 | 4 |
| Comp. Ex. 1 | - | - | 100 | 7 | 3.3 | 130 | 16 | 92 | 2 | 1 | 4 | 4 |

The results in Table 2 indicate that the thiol (2a) was obtained in 27% yield in Example 10 even when the amount of the powdered sulfur (S₈) was changed from 3.3 molar equivalents to 1.7 molar equivalents in terms of S element. In Example 11, it was found that the thiol (2a) was obtained in 64% yield, similar to Example 1, even without zeolite. In Examples 12 to 13, it was found that the thiol (2a) was obtained in 54% or 18% yield even when zeolite was not used and the hydrogen pressure was changed from 7.0 MPa to 5.0 MPa or 3.0 MPa during preparation. Furthermore, in Examples 14 to 15, it was found that the thiol (2a) was obtained in 55% or 62% yield even when zeolite was not used and the reaction temperature was changed from 130 °C to 120 °C or 140 °C.

In contrast, it was found in Comparative Example 1 that, although the conversion rate was not so low as 92% without the catalyst, the yield of the thiol (2a) was as low as 2% and the ratio of the yield of the thiol (2a) to the total yield of the thiol (2a) and dialkyl monosulfides (3a) to (5a) was as low as 18.1%.

### (Example 16)

After charging a stirrer, 6 mmol of the compound (1a) (H₂C=C-C₁₂H₂₅), 0.4125 molar equivalents of the powdered sulfur (S₈) (3.3 molar equivalents as S element), and 6.6 mol% of CoOₓ-SiO_{y} into an autoclave, hydrogen was pressure-charged until reaching 7.0 MPa. The temperature of the autoclave was raised to 130 °C while stirring at 800 rpm using a magnetic stirrer, and the reaction was carried out at 130 °C for a predetermined time (t). Then, after cooling to room temperature and opening a pressure valve, 20 mg tridecane (1.7 parts by mass with respect to 100 parts by mass of the compound (1a)) was added to the reaction solution as an internal standard. Then, air was blown in to remove any residual hydrogen sulfide. Unreacted sulfur and Co₃O₄ were removed by a centrifugation separator to obtain the thiol (2a). The temporal change in the yield of the thiol (2a) and the total yield of the dialkyl monosulfides (3a) to (5a) in Example 16 are illustrated in FIG. 3.

As illustrated in FIG. 3, it was found that almost all of the compound (1a) reacted after elapse of about 3 hours, where the conversion rate was maximized after about 6 hours. The reduction reaction of the thiol (2a) started after about 6 hours and the yield of the thiol (2a) was found to be maximized after about 9 hours. The yields of the dialkyl monosulfides (3a) to (5a) were found to be almost unchanged after about 3 hours.

### (Evaluation of Catalyst Properties)

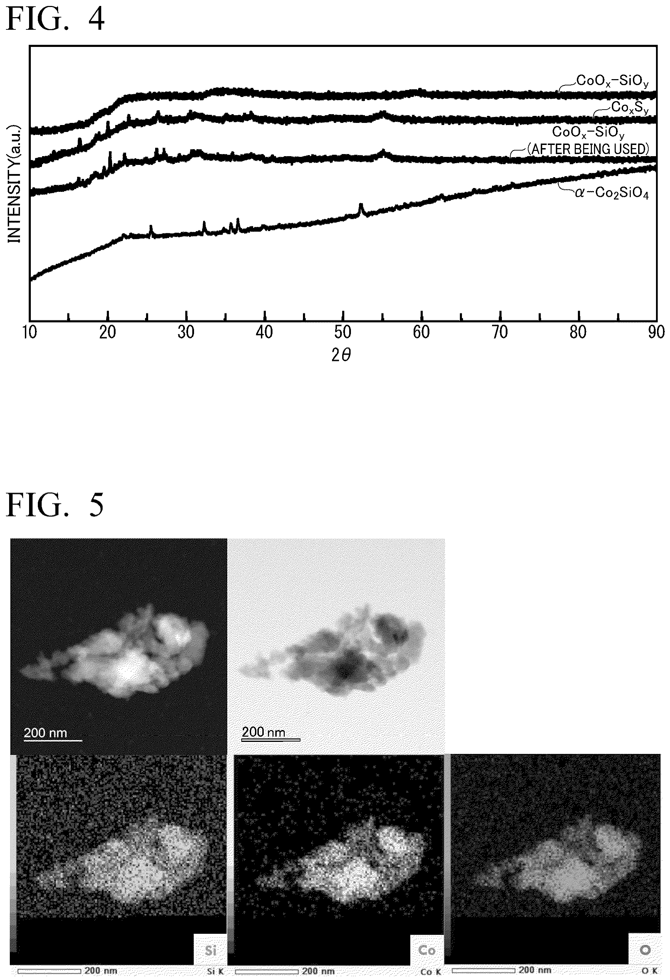

XRD measurements were performed on CoS and CoS₂ before being used in Examples 2 and 3, CoOₓ-SiO_{y} before being used in Example 6, CoOₓ-SiO_{y} after being used in Example 6, and α-Co₂SiO₄ before being used in Example 9 using an X-ray diffractometer ("MiniFlex 600" manufactured by Rigaku Holdings Corporation). XRD measurements were performed under the following conditions. The results are illustrated in FIG. 4.

### [XRD Measurement Conditions]

Cu Kα-ray source used (λ = 0.15418 nm)
Measurement speed: 1.5°/min

As illustrated in FIG. 4, when CoOₓ-SiO_{y} was used as a catalyst in the synthesis reaction of the thiol (2a) in Example 6, it was found that its crystal structure changed to one similar to that of CoₓS_{y} used in Examples 2 and 3 and different from that of α-Co₂SiO₄ used in Example 9.

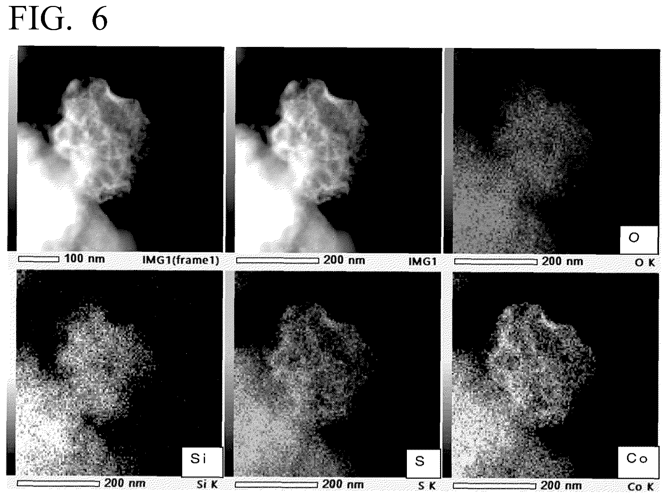

Next, elemental analysis was performed on CoOₓ-SiO_{y} before being used in Example 6 and CoOₓ-SiO_{y} after being used in Example 6 using an energy dispersive X-ray spectrometer (EDS) (manufactured by JEOL Ltd., product name "JEM-ARM200F"). EDS measurements were performed under the following conditions. Results are illustrated in FIGS. 5 and 6.

### [EDS Measurement Conditions]

Room temperature, high vacuum conditions
Data import time: 20 mmsec
Resolution 128 × 128

As illustrated in FIG. 5, in CoOₓ-SiO_{y} before being used, cobalt (Co), silica (Si), and oxygen (O) were each found to be almost uniformly distributed all over the catalyst. As illustrated in FIG. 6, in CoOₓ-SiO_{y} after being used, sulfur (S) was almost uniformly distributed all over the catalyst while the respective distributions of cobalt (Co), silica (Si) and oxygen (O) were maintained.

A plurality of samples of CoOₓ-SiO_{y} after being used in Example 6 were prepared, in which organic substance was oxidized using sulfuric acid and royal water to extract cobalt (Co) in an aqueous solution. The cobalt content, which was measured in the resulting aqueous solution, was less than 0.09 ppm in all of the samples. From these results, it was confirmed that the cobalt (Co) contained in CoOₓ-SiO_{y} was hardly eluted in the synthesis reaction and that the catalytic activity inherent to the catalyst could be maintained in the present manufacturing method.

### (Examples 18 to 35)

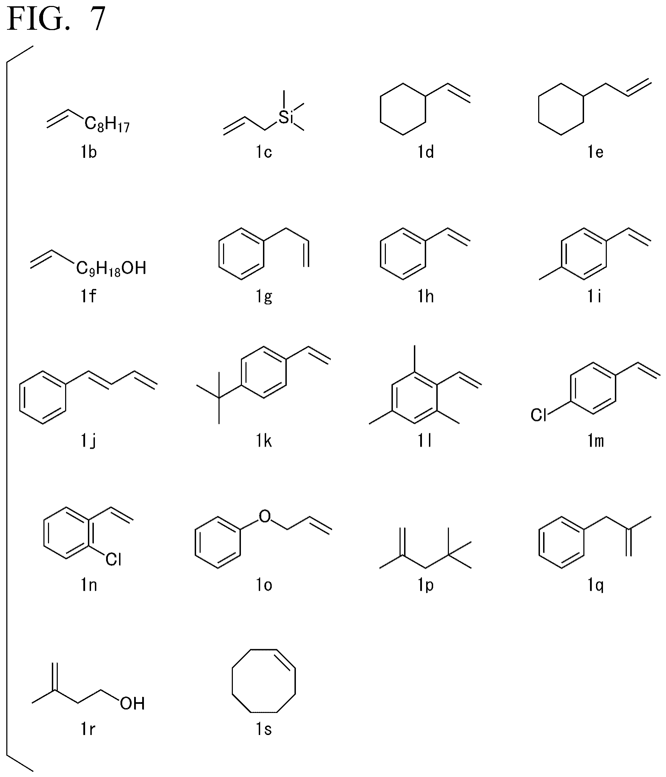

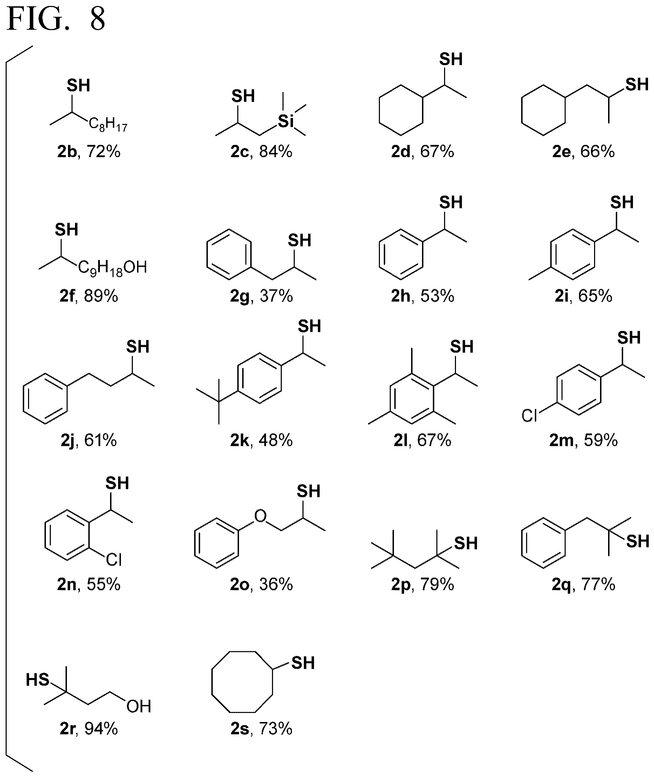

Thiols (2b) to (2s) illustrated in FIG. 8 were synthesized in the same manner as in Example 6 except that the compound (1a) (H₂C=C-C₁₂H₂₅) was respectively replaced with compounds (1b) to (1s) illustrated in FIG. 7. The respective yields of the obtained thiols (2b) to (2s) are illustrated in FIG. 8.

As illustrated in FIG. 8, it was found that the reaction of any of the compounds (1b) to (1s) with powdered sulfur in the presence of hydrogen, CoOₓ-SiO_{y}, and zeolite in any of Examples 18 to 35 produced the thiols (2b) to (2s) at yields of 36% to 94%.

### Industrial Applicability

The above thiol production method, in which thiol can be produced without requiring the difficult-to-handle hydrogen sulfide gas and expensive organic base and without requiring UV irradiation equipment, platinum electrodes, and the like with the use of hydrogen gas and catalysts that are relatively easy to be obtained and handled, is extremely useful in that thiol can be easily produced in various regions.

## Claims

1. A thiol production method comprising reacting an alkene (1) or a derivative of the alkene (1) with sulfur in a presence of hydrogen and a metallic element to obtain a thiol (2).

2. The thiol production method according to claim 1, wherein a pressure of supplied hydrogen is in a range from 0.1 MPa to 10 MPa inclusive when the alkene (1) or the derivative of the alkene (1) is reacted with sulfur.

3. The thiol production method according to claim 1 or 2, wherein a heating temperature is in a range from 100 °C to 200 °C inclusive when the alkene (1) or the derivative of the alkene (1) is reacted with sulfur in the presence of hydrogen.

4. The thiol production method according to claim 1, wherein the metallic element is one or more metallic elements selected from Group 6 to Group 11.

5. The thiol production method according to claim 4, wherein the metallic element is a metallic element constituting a metal oxide or a metal sulfide.

6. The thiol production method according to claim 1, wherein an added amount of the metallic element with respect to the alkene (1) or the derivative of the alkene (1) is in a range from 0.1 mol% to 10 mol% inclusive.

7. The thiol production method according to claim 1, wherein the alkene (1) or the derivative of the alkene (1) is reacted with sulfur in the presence of hydrogen, the metallic element, and zeolite.

8. The thiol production method according to claim 7, wherein the zeolite is basic.

9. The thiol production method according to claim 8, wherein the zeolite has a type X or type A pore structure.

10. The thiol production method according to claim 7, wherein an added amount of the zeolite is in a range from 2.0 parts by mass to 25 parts by mass inclusive with respect to 100 parts by mass of the alkene (1).

11. The thiol production method according to claim 1, wherein the alkene (1) is represented by R¹R²C=CH₂ ... (A), where R¹ is an alkyl group, R² is a hydrogen atom or an alkyl group, and a total number of carbon atoms of R¹ and R² is 2 to 20.

12. The thiol production method according to claim 11, wherein the total number of the carbon atoms of R¹ and R² in the above formula (A) is 2 to 16.
